# EUROPEAN PATENT APPLICATION

(11) **EP 3 141 917 A1**
(43) Date of publication of application: **15.03.2017**
(21) Application number: 15789135.9
(22) Date of filing: 07.05.2015
(51) Int. Cl.: G01R 31/02

(54) **APPARATUS FOR SENSING COIN TYPE BATTERIES OF PORTABLE MEDICAL DEVICE**

(30) Priority: 07.05.2014 KR 20140054088
(71) Applicant: i-Sens, Inc., Seoul 137-873 (KR)
(72) Inventor: LEE, Jin Won, Seoul 133-751 (KR); RYU, Chang Woo, Seoul 136-777 (KR); CHA, Geun Sig, Seoul 120-841 (KR); NAM, Hak hyun, Seoul 142-742 (KR)
(74) Representative: Groth & Co. KB
(86) International application number: PCT/KR2015/004557
(87) International publication number: WO 2015/170893

(57) **Abstract**

The present invention relates to an apparatus for sensing coin type batteries of a portable medical device. In a portable medical device which uses a plurality of coin type batteries, it is possible to sense whether or not all of the plurality of coin type batteries are correctly mounted, and to accurately measure biological values such as blood sugar, etc. by operating only in the case that all of the plurality of coin type batteries are correctly mounted.

## Description

### TECHNICAL FIELD

The present disclosure relates to an apparatus for sensing coin-type batteries in a portable medical device. More particularly, the present disclosure relates to an apparatus for sensing coin-type batteries in a portable medical device, the apparatus being able to sense whether or not a plurality of coin-type batteries are ordinarily accommodated in a portable medical device using the plurality of coin-type batteries and being configured to accurately measure biometric values, such as a level of blood glucose, by only operating when all batteries among the plurality of coin-type batteries are ordinarily accommodated.

### BACKGROUND ART

Coin-type batteries are also commonly referred to as CR-type batteries or button-type batteries. Coin-type batteries have the shape of a coin or a button having a positive contact and a negative contact. Portable medical devices, such as a blood glucose self-monitoring systems, use small and light coin-type batteries to improve the portability thereof. A housing in which coin-type batteries are accommodated has negative terminals and positive terminals with which the negative poles and positive poles of the coin-type batteries are in contact.

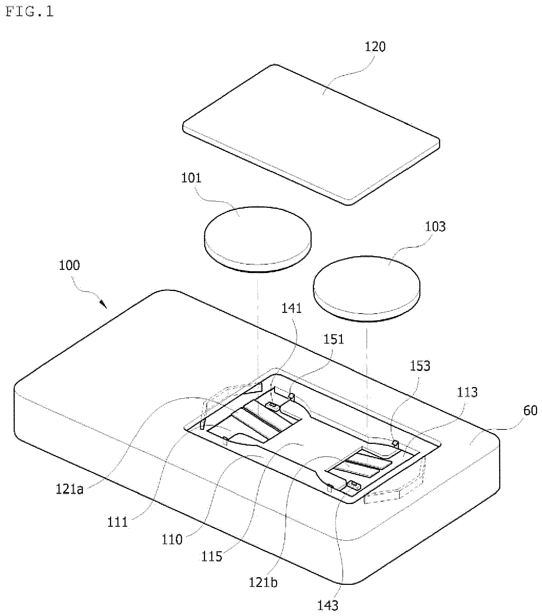

FIG. 1 is an exploded perspective view schematically illustrating a housing of a portable medical device of the related art, with coin-type batteries being accommodated in the housing.

Referring to FIG. 1, the coin-type battery-accommodating housing 10 of the related art includes: a receptacle 13 having openings formed in a predetermined position in a portable medical device 1; a printed circuit board (PCB) 15 disposed within the receptacle 13; positive (+) terminals 16 and negative (-) terminals disposed on the PCB 15, and a battery cover 19 covering accommodated coin-type batteries. The positive terminals 16 and the negative terminals 17 are exposed through the openings of the receptacle 13 and are electrically connected to coin-type batteries 20 and 21 seated thereon to be supplied with electricity from the batteries 20 and 21.

The positive terminals 16 and the negative terminals 17 are disposed on the PCB 15 to protrude through the openings of the receptacle 13. The positive terminals 16 and the negative terminals 17 are electrically connected to the PCB 15 by, for example, soldering.

The positive terminals 16 and the negative terminals 17 form seating recesses in which the coin-type batteries 20 are seated. The positive terminals 16 form side portions of the recesses to be come into contact with the positive (+) poles of the coin-type batteries 20, while the negative terminals 17 form the bottom surfaces of the recesses to come into contact with the negative (-) poles of the coin-type batteries 20. The contours of the positive terminals 16 conform to the contours of the coin-type batteries.

In the coin-type battery housing of related-art as described above, the contact between the coin-type battery and the positive terminal or the negative terminal may sometimes become unstable due to impacts or shaking applied to the portable medical device. A portable medical device, such as a blood glucose self-monitoring system, has information regarding a history of measurement of the blood glucose of a user stored in an internal memory thereof. Thus, information regarding the history of measurement of the blood glucose of a user must be safely protected. During the measurement of blood glucose, the supply of power is required to be reliable, so that blood glucose can be accurately measured.

In a portable medical device using at least two coin-type batteries, the entirety of coin-type batteries must be accommodated ordinarily in the portable medical device. When specific batteries among the plurality of coin-type batteries are not ordinarily accommodated, the portable medical device may not be reliably supplied with power even though the portable medical device may be able to operate. Then, it is difficult to accurately measure biometric values using the portable medical device. The power of the coin-type batteries may be wasted, since the power of the specific batteries among the plurality of batteries may be consumed in a one-sided manner.

However, it may be difficult for actual users to visually determine whether or not all batteries among a plurality of batteries are ordinarily accommodated in a portable medical device. It may also be difficult to find a poor contact between coin-type batteries and the negative or positive terminals.

### DISCLOSURE

### Technical Problem

Accordingly, the present disclosure has been made in consideration of the above-described problems occurring in the related-art portable medical devices having coin-type batteries as described above, and the present disclosure proposes an apparatus for sensing coin-type batteries in a portable medical device, the apparatus being able to sense whether or not a plurality of coin-type batteries are ordinarily accommodated in a portable medical device using the plurality of coin-type batteries.

The present disclosure also proposes an apparatus for sensing coin-type batteries in a portable medical device, the apparatus being configured to operate only when all batteries among the plurality of coin-type batteries are ordinarily mounted, thereby being able to accurately measure biometric values, such as a level of blood glucose.

### Technical Solution

According to an aspect of the present disclosure, an apparatus for sensing coin-type batteries disposed in a portable medical device may include: a battery-accommodating housing in which a first coin-type battery and a second coin-type battery are accommodated; a terminal part including a negative terminal configured to be in contact with a negative pole of the first coin-type battery or a negative pole of the second coin-type battery and a positive terminal configured to be in contact with a positive pole of the first coin-type battery or a positive pole of the second coin-type battery; a contact sensing part including a first coin-type battery contact sensor coming into contact with the negative pole of the first coin-type battery when the first coin-type battery is accommodated in the battery-accommodating housing and a second coin-type battery contact sensor coming into contact with the negative pole of the second coin-type battery when the second coin-type battery is accommodated in the battery-accommodating housing; and a controller supplying electric power from the first con-type battery and the second con-type battery to a portable medical device when the first coin-type battery contact sensor is in contact with the negative pole of the first coin-type battery and the second coin-type battery contact sensor is in contact with the negative pole of the second coin-type battery.

The controller may include: a first switch being controlled to be turned on to supply electric power from the first coin-type battery to the portable medical device when the first coin-type battery contact sensor is in contact with the negative pole of the first coin-type battery; and a second switch being controlled to be turned on to supply electric power from the second coin-type battery to the portable medical device when the second coin-type battery contact sensor is in contact with the negative pole of the second coin-type battery. When the first coin-type battery contact sensor is in contact with the negative pole of the first coin-type battery and the second coin-type battery contact sensor is not in contact with the negative pole of the second coin-type battery, the second switch controls the supply of electric power to the electronic elements of the portable medical device to be stopped, and when the second coin-type battery contact sensor is in contact with the negative pole of the second coin-type battery and the first coin-type battery contact sensor is not in contact with the negative pole of the first coin-type battery, the first switch controls the supply of electric power to the electronic elements of the portable medical device to be stopped.

Each of the first switch and the second switch may be a switch device, wherein the switch device is controlled to be turned on when the first coin-type battery contact sensor is in contact with the negative pole of the first coin-type battery or when the second coin-type battery contact sensor is in contact with the negative pole of the second coin-type battery. It is preferable that the first switch or the second switch is one selected from the group consisting of a metal oxide silicon field effect transistor (MOSFET), a bipolar junction transistor (BJT), and a field effect transistor (FET).

The first coin-type battery contact sensor may be disposed in a range in which the first coin-type battery contact sensor protrudes upwardly from a bottom surface of the battery-accommodating housing to come into contact with the negative pole of the first coin-type battery when the first coin-type battery is accommodated in the battery-accommodating housing. The second coin-type battery contact sensor is disposed in a range in which the second coin-type battery contact sensor protrudes upwardly from a bottom surface of the battery-accommodating housing to come into contact with the negative pole of the second coin-type battery when the second coin-type battery is accommodated in the battery-accommodating housing.

It is preferable that the apparatus for sensing coin-type batteries may further include: a first fixing portion holding the negative pole of the first coin-type battery to be in contact with the first coin-type battery contact sensor when the first coin-type battery is accommodated in the battery-accommodating housing; and a second fixing portion holding the negative pole of the second coin-type battery to be in contact with the second coin-type battery contact sensor when the second coin-type battery is accommodated in the battery-accommodating housing.

### Advantageous Effects

The apparatus for sensing coin-type batteries in a portable medical device according to the present disclosure has the following effects.

The apparatus for sensing coin-type batteries in a portable medical device according to the present disclosure can sense whether or not a plurality of coin-type batteries are ordinarily accommodated in a portable medical device using the plurality of coin-type batteries, thereby easily detecting a poor contact between the coin-type batteries and the negative or positive terminals of the portable medical device.

In addition, the apparatus for sensing coin-type batteries in a portable medical device according to the present disclosure is configured to only operate when all batteries among the plurality of coin-type batteries are ordinarily mounted, thereby accurately measuring biometric values, such as a level of blood glucose.

### DESCRIPTION OF DRAWINGS

FIG. 1 is an exploded perspective view schematically illustrating a housing of a portable medical device of the related art, with coin-type batteries being accommodated in the housing;
FIG. 2 is an exploded perspective view illustrating an apparatus for sensing coin-type batteries in a portable medical device according to the present invention;
FIG. 3 is a view schematically illustrating the housing according to the present invention;
FIG. 4 is a functional block diagram illustrating a controller according to the present invention;
FIG. 5 is a circuit diagram view illustrating a specific example of the controller according to the present invention; and
FIG. 6 illustrates a position in which coin-type batteries are accommodated in the housing according to the present invention.

### MODE FOR INVENTION

Hereinafter, reference will be made to an apparatus for sensing coin-type batteries in a portable medical device according to the present disclosure in detail, examples of which are illustrated in the accompanying drawings and described below.

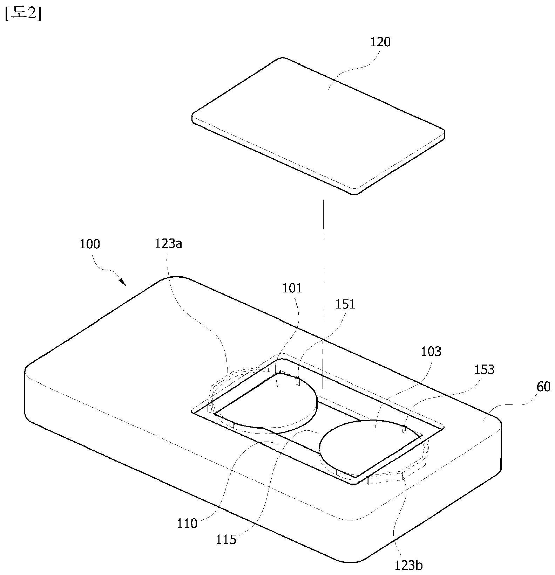

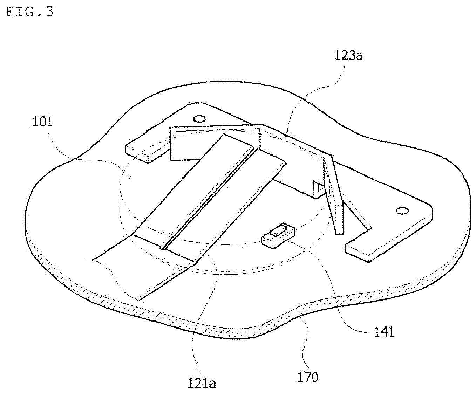

FIG. 2 is an exploded perspective view illustrating an apparatus for sensing coin-type batteries in a portable medical device according to the present invention, and FIG. 3 is a view schematically illustrating the housing according to the present invention.

Referring to FIG. 2 and FIG. 3, a portable medical device 100, such as a blood glucose self-monitoring system, has a battery-accommodating housing 110 in which a plurality of, for example, two coin-type batteries 101 and 103 are accommodated. The battery-accommodating housing 110 is opened and closed using a cover 120, and a printed circuit board (PCB) 170 is disposed on the bottom surface of the battery-accommodating housing 110. Electronic elements for controlling the operation of the portable medical device are disposed on the PCB 170. In particular, the PCB is provided with a control unit that controls required power to only be supplied to the portable medical device when a first coin-type battery 101 and a second coin-type battery 103 are ordinarily accommodated therein.

The battery-accommodating housing 110 has seating recesses 111 and 113 in which the coin-type batteries 101 and 103 are seated, with a support 115 supporting the two coin-type batteries being disposed between the seating recesses 111 and 113. The first coin-type battery 101 of the two coin-type batteries is supported by the support 115 to be disposed in the seating recess 111, while the second first coin-type battery 103 of the two coin-type batteries is supported by the support 115 to be disposed in the seating recess 113.

A first coin-type battery contact sensor 141 and a second coin-type battery contact sensor 143 are disposed on the PCB 170. The first coin-type battery contact sensor 141 protrudes upwardly from the seating recess 111, while the second coin-type battery contact sensor 143 protrudes upwardly from the seating recess 113. Thus, the first coin-type battery contact sensor 141 comes into contact with the negative pole of the first coin-type battery 101 when the first coin-type battery 101 is accommodated in the battery-accommodating housing 110, while the second coin-type battery contact sensor 143 comes into contact with the negative pole of the second coin-type battery 103 when the second coin-type battery 103 is accommodated in the battery-accommodating housing 110.

A portable medical device housing 60 is disposed at a predetermined distance from the top surface of the battery-accommodating housing 110, thereby defining accommodation spaces 130a and 130b between the battery-accommodating housing 110 and the portable medical device housing 60, such that the first coin-type battery 101 and the second coin-type battery 103 are accommodated in the accommodation spaces 130a and 130b. A positive terminal 123a for the first coin-type battery 101 is disposed in the accommodation space 130a to be in close contact with the positive pole of the first coin-type battery 101, while a positive terminal 123b for the second coin-type battery 103 is disposed in the accommodation space 130b to be in close contact with the positive pole of the second coin-type battery 103.

Thus, when the first coin-type battery 101 is disposed in the accommodation space 130a, the portable medical device housing 60 supports the first coin-type battery 101 to be closely fixed to a negative terminal 121a for the first coin-type battery or the first coin-type battery contact sensor 141. In the same manner, when the second coin-type battery 103 is disposed in the accommodation space 130b, the portable medical device housing 60 supports the second coin-type battery 103 to be closely fixed to a negative terminal 121b for the second coin-type battery or the second coin-type battery contact sensor 143.

Describing the position in which the coin-type batteries are accommodated in the battery-accommodating housing in greater detail, the negative terminal 121a for the first coin-type battery and the negative terminal 121b for the second coin-type battery, mounted on the PCB (not shown) to protrude through the seating recess 111 and the seating recess 113, are disposed on the bottom surface of the support 115. It is preferable that the negative terminal 121a for the first coin-type battery and the negative terminal 121b for the second coin-type battery are implemented as integral structure formed of an elastic material, both ends of which protrude upwardly through the seating recess 111 and the seating recess 113. When the first coin-type battery 101 or the second coin-type battery 103 is seated in the seating recess 111 or 113, the negative terminal 121a or 121b comes into close contact with the negative pole formed on the bottom end of the first coin-type battery 101 or the second coin-type battery 103.

The positive terminal 123a for the first coin-type battery, mounted on the PCB, is disposed in the accommodation space 130a, while the positive terminal 123b for the second coin-type battery, mounted on the PCB, is disposed in the accommodation space 130b. The side surface of the first coin-type battery 101 forms the positive pole, which comes into contact with the positive terminal 123a for the first coin-type battery when the first coin-type battery 101 is seated in the seating recess 111. The side surface of the second coin-type battery 103 forms the positive pole, which comes into contact with the positive terminal 123b for the second coin-type battery when the second coin-type battery 103 is seated in the seating recess 113.

When the first coin-type battery 101 is accommodated in the accommodation space 130a, the portable medical device housing 60 presses and supports the first coin-type battery 101, which moves upwardly due to the elasticity of the negative terminal 121a for the first coin-type battery, in a downward direction so that the first coin-type battery 101 is closely fixed to the negative terminal 121a for the first coin-type battery or the first coin-type battery contact sensor 141. In the same manner, when the second coin-type battery 103 is accommodated in the accommodation space 130b, the portable medical device housing 60 presses and supports the second coin-type battery 103, which moves upwardly due to the elasticity of the negative terminal 121b for the second coin-type battery, in a downward direction so that the second coin-type battery 103 is closely fixed to the negative terminal 121b for the second coin-type battery or the second coin-type battery contact sensor 143.

The first coin-type battery contact sensor 141 and the second coin-type battery contact sensor 143 are mounted on the PCB. Here, the first coin-type battery contact sensor 141 is disposed to protrude upwardly through the seating recess 111 to come into contact with the negative pole of the first coin-type battery 101 when the first coin-type battery 101 is accommodated in the battery-accommodating housing 110. The second coin-type battery contact sensor 143 is disposed to protrude upwardly through the seating recess 113 to come into contact with the negative pole of the second coin-type battery 103 when the second coin-type battery 103 is accommodated in the battery-accommodating housing 110. It is preferable that a first fixing portion 151 and a second fixing portion 153 are disposed on the top portions of the battery-accommodating housing 110. When the first coin-type battery 101 is accommodated in the battery-accommodating housing, the first fixing portion 151 presses the first coin-type battery 101 in the direction of the bottom of the battery-accommodating housing 110 so that the first coin-type battery 101 is closely held. When the second coin-type battery 103 is accommodated in the battery-accommodating housing, the second fixing portion 153 presses the second coin-type battery 103 in the direction of the bottom of the battery-accommodating housing 110 so that the second coin-type battery 103 is closely held.

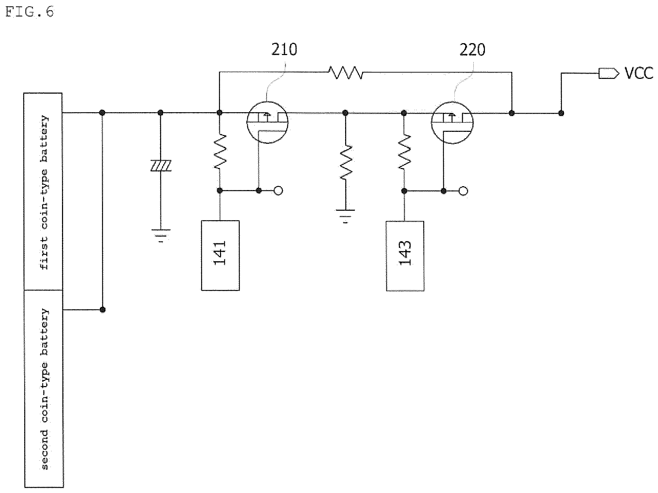

As illustrated in FIG. 6, when the first coin-type battery 101 is accommodated in the battery-accommodating housing 110, the first fixing portion 151 brings the negative pole of the first coin-type battery 101 into close contact with the first coin-type battery contact sensor 141, together with the portable medical device housing 60 of the accommodation space 130a. When the second coin-type battery 103 is accommodated in the battery-accommodating housing 110, the second fixing portion 153 brings the negative pole of the second coin-type battery 103 into close contact with the second coin-type battery contact sensor 143, together with the portable medical device housing 60 of the accommodation space 103b.

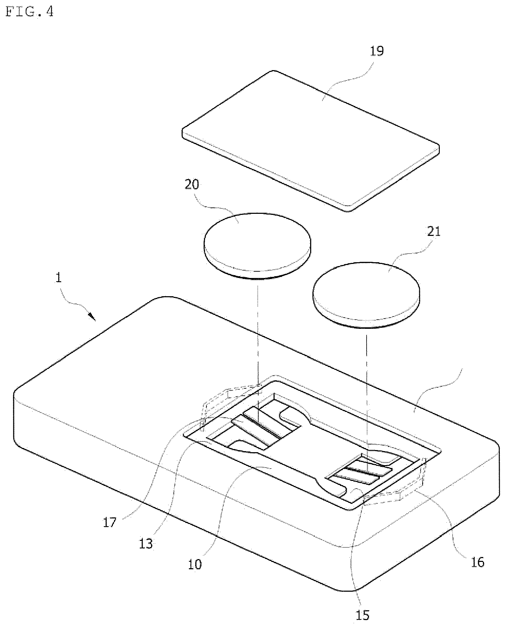

FIG. 4 is a functional block diagram illustrating a controller according to the present invention.

Described in greater detail with reference to FIG. 4, the controller 200 controls the supply of electric power to the portable medical device from the first coin-type battery 101 and the second coin-type battery 103 when the first coin-type battery contact sensor 141 is in contact with the negative pole of the first coin-type battery 101 and the second coin-type battery contact sensor 143 is in contact with the negative pole of the second coin-type battery 103. The controller 200 includes a first switch 210 and a second switch 220. The first switch 210 is controlled to be turned on to supply electric power to the electronic elements of the portable medical device from the first coin-type battery 101 when the first coin-type battery contact sensor 141 is in contact with the negative pole of the first coin-type battery 101. The second switch 220 is controlled to be turned on to supply electric power to the electronic elements of the portable medical device from the second coin-type battery 103 when the second coin-type battery contact sensor 143 is in contact with the negative pole of the second coin-type battery 103.

When the second coin-type battery contact sensor 143 is in contact with the negative pole of the second coin-type battery 103 and the first coin-type battery contact sensor 141 is not in contact with the negative pole of the first coin-type battery 101, the first switch 210 controls the supply of electric power to the electronic elements of the portable medical device to be stopped. When the first coin-type battery contact sensor 141 is in contact with the negative pole of the first coin-type battery 101 and the second coin-type battery contact sensor 143 is not in contact with the negative pole of the second coin-type battery 103, the second switch 220 controls the supply of electric power to the electronic elements of the portable medical device to be stopped.

That is, when only the second coin-type battery 103 is ordinarily accommodated and the first coin-type battery 101 is not accommodated or is not ordinarily accommodated, the second coin-type battery contact sensor 143 is in contact with the negative pole of the second coin-type battery 103 and the first coin-type battery contact sensor 141 is not in contact with the negative pole of the first coin-type battery 101, so that the first switch 210 is turned off and the second switch 200 is turned on to control the supply of electric power to the electronic elements of the portable medical device to be stopped. In addition, when only the first coin-type battery 101 is ordinarily accommodated and the second coin-type battery 103 is not accommodated or is not ordinarily accommodated, the first coin-type battery contact sensor 141 is in contact with the negative pole of the first coin-type battery 101 and the second coin-type battery contact sensor 143 is not in contact with the negative pole of the second coin-type battery 103, so that the first switch 210 is turned on and the second switch 200 is turned off to control the supply of electric power to the electronic elements of the portable medical device to be stopped.

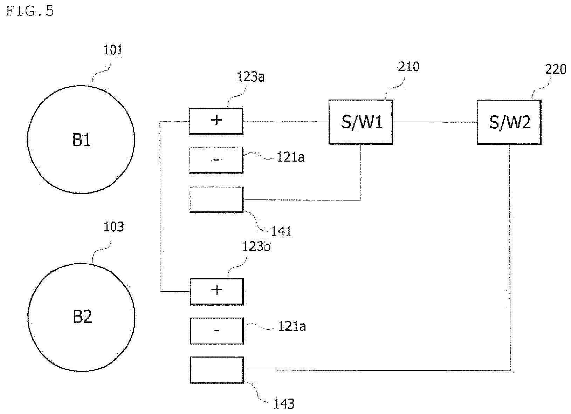

FIG. 5 is a circuit diagram view illustrating a specific example of the controller according to the present invention.

Referring to FIG. 5, the first switch 210 is a field effect transistor (FET), the gate terminal of which is connected to the first coin-type battery contact sensor 141. When the negative pole of the first coin-type battery 101 is in contact with the first coin-type battery contact sensor 141, the gate of the first switch 210 is dropped to a low level, so that the first switch 210 is turned on. In addition, the second switch 220 is an FET, the gate terminal of which is connected to the second coin-type battery contact sensor 143. When the negative pole of the second coin-type battery 103 is in contact with the second coin-type battery contact sensor 143, the gate of the second switch 220 is dropped to a low level, so that the first switch 210 is turned on. Consequently, when both the first coin-type battery 101 and the second coin-type battery 103 are sensed to be ordinarily accommodated by the first coin-type battery contact sensor 141 and the second coin-type battery contact sensor 143, both the first switch 210 and the second switch 220 are turned on to supply electric power to the electronic elements of the portable medical device from the first coin-type battery 101 and the second coin-type battery.

While the present disclosure has been described with reference to the certain exemplary embodiments shown in the drawings, these embodiments are illustrative only. Rather, it will be understood by a person skilled in the art that various modifications and equivalent other embodiments may be made therefrom. Therefore, the true scope of the present disclosure shall be defined by the concept of the appended claims.

## Claims

1. An apparatus for sensing coin-type batteries disposed in a portable medical device, the apparatus comprising:
a battery-accommodating housing in which a first coin-type battery and a second coin-type battery are accommodated;
a terminal part comprising a negative terminal configured to be in contact with a negative pole of the first coin-type battery or a negative pole of the second coin-type battery and a positive terminal configured to be in contact with a positive pole of the first coin-type battery or a positive pole of the second coin-type battery;
a contact sensing part comprising a first coin-type battery contact sensor coming into contact with the negative pole of the first coin-type battery when the first coin-type battery is accommodated in the battery-accommodating housing and a second coin-type battery contact sensor coming into contact with the negative pole of the second coin-type battery when the second coin-type battery is accommodated in the battery-accommodating housing; and
a controller supplying electric power from the first con-type battery and the second con-type battery to a portable medical device when the first coin-type battery contact sensor is in contact with the negative pole of the first coin-type battery and the second coin-type battery contact sensor is in contact with the negative pole of the second coin-type battery.

2. The apparatus according to claim 1, wherein the controller comprises:
a first switch being controlled to be turned on to supply electric power from the first coin-type battery to the portable medical device when the first coin-type battery contact sensor is in contact with the negative pole of the first coin-type battery; and
a second switch being controlled to be turned on to supply electric power from the second coin-type battery to the portable medical device when the second coin-type battery contact sensor is in contact with the negative pole of the second coin-type battery,
wherein, when the first coin-type battery contact sensor is in contact with the negative pole of the first coin-type battery and the second coin-type battery contact sensor is not in contact with the negative pole of the second coin-type battery, the second switch controls the supply of electric power to the electronic elements of the portable medical device to be stopped, and when the second coin-type battery contact sensor is in contact with the negative pole of the second coin-type battery and the first coin-type battery contact sensor is not in contact with the negative pole of the first coin-type battery, the first switch controls the supply of electric power to the electronic elements of the portable medical device to be stopped.

3. The apparatus according to claim 2, wherein each of the first switch and the second switch is a field effect transistor, wherein the field effect transistor is controlled to be turned on when the first coin-type battery contact sensor is in contact with the negative pole of the first coin-type battery or when the second coin-type battery contact sensor is in contact with the negative pole of the second coin-type battery.

4. The apparatus according to claim 2, wherein the first coin-type battery contact sensor is disposed to protrude upwardly from a bottom surface of the battery-accommodating housing to come into contact with the negative pole of the first coin-type battery when the first coin-type battery is accommodated in the battery-accommodating housing, and the second coin-type battery contact sensor is disposed to protrude upwardly from a bottom surface of the battery-accommodating housing to come into contact with the negative pole of the second coin-type battery when the second coin-type battery is accommodated in the battery-accommodating housing.

5. The apparatus according to claim 2, further comprising:
a first fixing portion holding the negative pole of the first coin-type battery to be in contact with the first coin-type battery contact sensor when the first coin-type battery is accommodated in the battery-accommodating housing; and
a second fixing portion holding the negative pole of the second coin-type battery to be in contact with the second coin-type battery contact sensor when the second coin-type battery is accommodated in the battery-accommodating housing.
